(19) Europäisches Patentamt
European Patent Office
Office européen des brevets

(11) Veröffentlichungsnummer: **0 154 885**
**B1**

(12) # EUROPÄISCHE PATENTSCHRIFT

(45) Veröffentlichungstag der Patentschrift: **18.01.89**

(51) Int. Cl.⁴: **C 07 D 253/06**, A 61 K 31/53

(21) Anmeldenummer: **85102133.7**

(22) Anmeldetag: **27.02.85**

(54) **Substituierte 2-Phenyl-hexahydro-1,2,4-triazin-3,5-dione, Verfahren zu ihrer Herstellung, ihre Verwendung als Tierarzneimittel und zur Bekämpfung von Protozoen.**

(30) Priorität: **12.03.84 DE 3408924**

(43) Veröffentlichungstag der Anmeldung:
**18.09.85 Patentblatt 85/38**

(45) Bekanntmachung des Hinweises auf die Patenterteilung:
**18.01.89 Patentblatt 89/3**

(84) Benannte Vertragsstaaten:
**AT BE CH DE FR GB IT LI LU NL SE**

(56) Entgegenhaltungen:
**EP-A-0 058 534**
**DE-A-2 449 645**
**DE-A-2 606 850**
**DE-A-2 722 537**

**CHEMICAL ABSTRACTS, Band 88, Nr. 21, 22. Mai 1978, Columbus, Ohio, USA. MINLIBAEVA, A.N.; BIGLOVA, R.Z.; SVETKIN, YU. V.: "Synthesis of 1,2,4-triazine derivatives by cyclization of 1-aryl-4-(chloro-acetyl)semicarbazides", Seite 606, Spalte 1, Zusammenfassung Nr. 152 571s & Org. Khim. 1976, 99-102**
**CHEMICAL ABSTRACTS, Band 92, Nr. 7, 18. Februar 1980, Columbus, Ohio, USA. MILLER, MAX W.; MYLARI, BANAVARA L.; HOWES, HAROLD L., JR.; LYNCH, MARTIN J.; KOCH, RICHARD C.: "Anticoccidial derivatives of 6-azauracil. 2. High potency and long**

(73) Patentinhaber: **HOECHST AKTIENGESELLSCHAFT, Postfach 80 03 20, D-6230 Frankfurt am Main 80 (DE)**

(72) Erfinder: **Rösner, Manfred, Dr., Unter den Buchen 7, D-6239 Eppstein/Taunus (DE)**
Erfinder: **Raether, Wolfgang, Dr., Falkensteinstrasse 6, D-6072 Dreieich (DE)**

(56) Entgegenhaltungen: (Fortsetzung)
**plasma life of N1-phenyl structures", Seite 20, Spalte 2, Zusammenfassung Nr 51 708u & J. Med. Chem. 1979, 22(12), 1483-7**
**CHEMICAL ABSTRACTS, Band 95, Nr. 21, 23. November 1981, Columbus, Ohio, USA. MILLER, MAX W.; MYLARI, BANAVARA L.; HOWES, HAROLD L., JR.; FIGDOR, SANFORD K.; LYNCH, MARTIN J.; LYNCH, JOHN E.; GUPTA, SHYAM K.; CHAPPEL, LARRY R.; KOCH, RICHARD C.: "Anticoccidial derivatives of 6-azauracil. 4. A 1000-fold enhancement of potency by phenyl sulfide and phenyl sulfone side chains", Seite 25, Spalte 2, Zusammenfassung Nr 180 651f & J. Med. Chem. 1981, 24(11), 1337-42**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Coccidiostatisch wirksame 1,2,4-Triazin-3,5-(2H,4H)-dione und ihre Herstellung sind u.a. aus der BE-PS 740403, DE-OS 2149645 [3][≙Chem. Abstr. 92 51708 (1980) und Chem. Abstr. 95 180561f (1981)], DE-OS 2423972 und DE-OS 2722537 = US-PS 4198407 bekannt. In der EP-A 58534 sind Hexahydro-1,2,4-triazin-3,5-dione mit einem Benzyl- oder Thienylmethylsubstituenten in 2-Stellung beschrieben. Ferner sind substituierte p-Aminophenyl-triazindione mit coccidiozider Wirkung aus DE-OS 2606850 bekannt.

Gegenstand der Erfindung sind neue substituierte 2-Phenylhexahydro-1,2,4-triazin-3,5-dione der allgemeinen Formel I

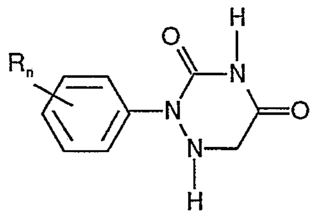

(I),

worin n = zwei oder drei und die einzelnen Substituenten R a) Wasserstoff, Chlor, $(C_1-C_6)$-Alkyl, $(C_1-C_6)$-Alkoxy, $(C_1-C_6)$-Alkylthio, $(C_1-C_6)$-Alkylsulfinyl oder $(C_1-C_6)$-Alkylsulfonyl oder b) einen jeweils $R_{a)}$-substituierten Phenoxy-, Phenylthio-, Phenylsulfinyl-, Phenylsulfonylrest bedeuten und wenigstens ein R ein vorstehend unter b) definierter Substituent ist, sowie deren Alkali-, Erdalkali- oder Ammoniumsalze.

Bevorzugt sind solche Verbindungen, in denen der Phenylrest in Formel I in 3,4,5-Stellung trisubstituiert ist.

Gegenstand der Erfindung ist weiterhin ein Verfahren zur Herstellung von substituierten Hexahydro-1,2,4-triazin-3,5-dionen der Formel I, dadurch gekennzeichnet, dass man ein substituiertes 1,2,4-Triazin-3,5-(2H,4H)-dion der allgemeinen Formel II

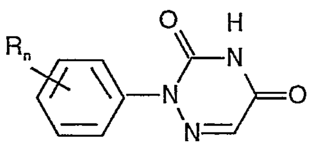

(II),

worin n und R die zu Formel I gegebenen Bedeutungen haben, selektiv an der C-N-Doppelbindung hydriert.

Die Reduktion kann nach üblichen Verfahren erfolgen, wie sie z.B. in Houben-Weyl, Methoden der Organischen Chemie, Band 4/1c S. 307ff und Band 4/1d S. 365ff beschrieben sind. Sie kann je nach den Gegebenheiten der Verbindung der Formel II z.B. durch katalytisch angeregten Wasserstoff mit Katalysatoren wie Raney-Nickel, Platin, Palladium, Platin-(IV)-oxid oder durch chemische Reduktion mit Metallen, Metallsalzen Metallcarbonylen oder komplexen Hydriden erfolgen. Beispielhaft seien genannt Natriumamalgam in Äthanol, Lithium in Ammoniak, Zinn-(II)chlorid in Salzsäure, Eisen in Eisessig, Lithiumaluminiumhydrid, Natriumborhydrid, Natriumcyanoborhydrid. Vorzugsweise erfolgt die Reduktion mit Zink in Eisessig oder Zinn-(II)chlorid in Salzsäure, gegebenenfalls in einem inerten Lösungs- oder Verdünnungsmittel wie Methanol, Äthanol, Toluol, Aceton, Butanon, Dimethoxyäthan, Tetrahydrofuran, Dioxan, Essigsäureäthylester, Pyridin, Eisessig. Man arbeitet im allgemeinen in einem Temperaturbereich von etwa 50°C bis 150°C, vorzugsweise zwischen 80°C und 120°C oder bei der Siedetemperatur des verwendeten Lösungsmittels oder Lösungsmittelgemisches.

Die Darstellungsverfahren für Verbindungen der allgemeinen Formel II sind literaturbekannt und z.B. in der DE-OS 2722537 = US-PS 4198407 beschrieben. Die Herstellung erfolgt zum Beispiel durch Diazotierung eines entsprechend substituierten Anilinderivates und Kupplung des Diazoniumsalzes mit N,N′-Bis(äthoxycarbonyl)malonsäurediamid mit anschliessender Cyclisierung, Verseifung und Decarboxylierung.

Die Verbindungen der Formel I können durch Zugabe von zweckmässig einem Moläquivalent Alkali, Erdalkali oder Ammoniak in die entsprechenden Salze übergeführt werden. Bevorzugt verwendet man dafür Natriumhydroxid, Natriummethylat, Natriumhydrid, Kaliumhydroxid, Calciumhydroxid, Calciumhydrid oder Ammoniak.

Die erfindungsgemässen Verbindungen der Formel I sind neue Chemotherapeutika, die gegen Protozoenerkrankungen insbesondere als Coccidiostatika verwendet werden können. Die Verbindungen sind ferner Zwischenprodukte zur Synthese von Arzneimitteln.

Die Coccidiose verursacht in der Geflügelhaltung Mortalität und damit grosse wirtschaftliche Verluste. Deshalb sind prophylaktische und therapeutische Massnahmen notwendig. Im Vordergrund steht die Prophylaxe, insbesondere die Verabreichung von Coccidiostatika im Futter, die den Ausbruch einer Coccidiose verhindert. Daneben können diese Mittel auch therapeutisch bei einer bereits vorliegenden Coccidiose eingesetzt werden.

Ein Coccidiostatikum muss gute Wirksamkeit gegen verschiedene Coccidienarten in niedrigen Anwendungskonzentrationen, gute Verträglichkeit und eine daraus resultierende grosse therapeutische Breite aufweisen. Daneben sollten neue Coccidiostatika gegen bereits arzneiresistente Coccidienstämme wirksam sein.

Die Verbindungen der Formel I und ihre Salze zeigen bereits in sehr geringen Mengen einen ausgeprägten Effekt gegen verschiedene Erreger der Coccidiose beim Geflügel und anderen Tierspezies bei gleichzeitiger sehr guter Verträglichkeit. Darüberhinaus beeinflussen sie mehrfacharzneimittel-resistente Erreger der Coccidiose.

Die Verbindungen der Formel I und ihre Salze können grundsätzlich als solche in Substanz verabreicht werden, insbesondere im Trinkwasser. Bevorzugt ist ihre Verwendung in Mischung mit geeignetem Trägermaterial.

Als Trägermaterial können die üblichen Futtermittelmischungen verwendet werden. Ein Wirkstoff der Formel I wird dabei dem Futter in einer Konzentration von 0,1–300 ppm, vorzugsweise 0,5–50 ppm, insbesondere 0,5–30 ppm zugemischt. Im Vergleich zu den bekannten Aralkyl-Hexahydrotriazinen der EP-A 58 534, die nur in einem Bereich von 70 bis 200 ppm im Futter oder Trinkwasser wirksam sind, zeichnen sich die erfindungsgemässen Verbindungen der Formel I und ihre Salze insbesondere dadurch aus, dass sie schon in wesentlich niedrigeren Konzentrationen wirksam sind und gleichzeitig eine gute Verträglichkeit haben. Es wurde gefunden, dass Verbindungen der Formel I im Ames-Test keine mutagene Wirkung zeigten.

Beispiel 1

2-[3,5-Dichlor-4-(4-methylthio-phenoxy)phenyl]hexahydro-1,2,4-triazin-3,5-dion

20 g 2-[3,5-Dichlor-4-(4-methylthio-phenoxy)phenyl]-1,2,4-triazin-3,5-(2H, 4H)-dion wurden in 250 ml Eisessig heiss gelöst. Man gab 30 g Zinkstaub portionsweise zu und erhitzte vier Stunden lang zum Rückfluss. Man filtrierte heiss ab, kochte den zinkhaltigen Rückstand noch einige Male mit Eisessig und/oder 2-Methoxyäthanol aus und filtrierte. Die gesammelten Lösungen wurden unter vermindertem Druck eingeengt und in Wasser eingerührt. Die entstandene Fällung wurde abgesaugt, mit Wasser gewaschen, getrocknet und aus Eisessig oder 2-Methoxyäthanol, ggf. unter Zusatz von Aktivkohle, umkristallisiert. Fp. 239 °C (aus 2-Methoxyäthanol).

NMR-Spektrum, 60 MHz, DMSO-$d_6$, TMS als innerer Standard, δ-Werte im ppm: $-NH-CH_2$-3,7 d, J=8 Hz, $-NH-CH_2$-6,53 tr, J=8HZ
IR-Spektrum, KBr: $-NH-CH_2$-3280 $cm^{-1}$, $-NH-CH_2$ 2910 $cm^{-1}$ C=O 1680, 1735 $cm^{-1}$

In analoger Verfahrensweise wurden jeweils aus den substituierten 1,2,4-Triazin-3,5-(2H, 4H)-dionen durch Reduktion die ebenso substituierten Hexahydro-1,2,4-triazin-3,5-dione erhalten:

Beispiel 2

2-[3,5-Dichlor-4-(4-methylsulfinyl-phenoxy)phenyl]hexahydro-1,2,4-triazin-3,5-dion, Fp. 234–235 °C Zers.

Beispiel 3

2-[3-Chlor-4-(4-methylthio-phenoxy)phenyl]hexahydro-1,2,4-triazin-3,5-dion, Fp. 209 °C

Beispiel 4

2-[3,5-Dimethyl-4-(4-methylthio-phenoxy)phenyl]hexahydro-1,2,4-triazin-3,5-dion, Fp. 193 °C

Beispiel 5

2-[4-(3-Methyl-4-methylthio-phenoxy)phenyl]hexahydro-1,2,4-triazin-3,5-dion, Fp. 168 °C

Beispiel 6

2-[3,5-Dichlor-4-(3-methyl-4-methylthio-phenoxy)phenyl]hexahydro-1,2,4-triazin-3,5-dion, Fp. 193 °C

Beispiel 7

2-(4-Phenylthio-phenyl)hexahydro-1,2,4-triazin-3,5-dion, Fp. 204–205 °C

Beispiel 8

2-[3,5-Dichlor-4-(4-chlorphenylthio)phenyl]hexahydro-1,2,4-triazin-3,5-dion, Fp. 247 °C Zers.

Beispiel 9

2-[4-(4-Methylthio-phenylthio)phenyl]hexahydro-1,2,4-triazin-3,5-dion, Fp. 222 °C

Beispiel 10

2-[3,5-Dichlor-4-(4-methylthio-phenylthio)phenyl]hexahydro-1,2,4-triazin-3,5-dion, Fp. 237–238 °C

Beispiel 11

2-[3,5-Dichlor-4-(4-methylthio-phenoxy)phenyl]hexahydro-1,2,4-triazin-3,5-dion

20 g 2-[3,5-Dichlor-4-(4-methylthio-phenoxy)phenyl]-1,2,4-triazin-3,5-(2H, 4H)-dion wurden in 200 ml Eisessig heiss gelöst. Man gab 25 g Zinn(II)chlorid · $2H_2O$ und anschliessend 100 ml konzentrierte Salzsäure zu und erhitzte anschliessend vier Stunden zum Rückfluss. Nach dem Abkühlen saugte man das Produkt ab, wusch mit Wasser neutral und kristallisierte aus 2-Methoxyäthanol um; Fp. 239 °C.

In analoger Verfahrensweise wurden jeweils aus den substituierten 1,2,4-Triazin-3,5-(2H, 4H)-dionen durch Reduktion die ebenso substituierten Hexahydro-1,2,4-triazin-3,5-dione der Beispiele 2 bis 10 erhalten.

Beispiel 12

2-[3,5-Dichlor-4-(4-methylsulfonyl-phenoxy)phenyl]hexahydro-1,2,4-triazin-3,5-dion

10 g 2-[3,5-Dichlor-4-(methylsulfonyl-phenoxy)phenyl]-1,2,4-triazin-3,5-(2H,4H)-dion werden in 150 ml Eisessig heiss gelöst, mit 10 g Zinkstaub versetzt und zwei Stunden zum Rückfluss erhitzt. Anschliessend wird heiss filtriert, und der zinkhaltige Rückstand dreimal mit 50 ml Eisessig ausgekocht. Die gesammelten Lösungen werden unter vermindertem Druck eingeengt, mit Wasser versetzt und abgesaugt.

Der verbleibende Feststoff wird aus Methanol unter Zusatz von Aktivkohle umkristallisiert, Fp. 240 °C Zers.

**Patentansprüche für die Vertragsstaaten: BE, CH, DE, FR, GB, IT, LI, LU, NL, SE**

1. Substituierte 2-Phenyl-hexahydro-1,2,4-triazin-3,5-dione der Formel

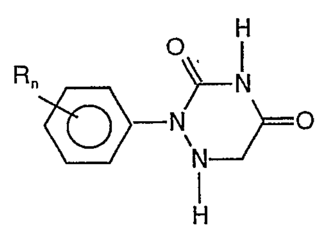

(I),

worin n = zwei oder drei und die einzelnen Substituenten
R = a) Wasserstoff, Chlor, $(C_1-C_6)$-Alkyl, $(C_1-C_6)$-Alkoxy, $(C_1-C_6)$-Alkylthio, $(C_1-C_6)$-Alkylsulfinyl, $(C_1-C_6)$-Alkylsulfonyl oder b) einen jeweils $R_{a)}$-substituierten Phenoxy-, Phenylthio-, Phenylsulfinyl- oder Phenylsulfonylrest bedeuten und wenigstens ein R ein vorstehend unter b) definierter Substituent ist, sowie deren Alkali-, Erdalkali- oder Ammoniumsalze.

2. Verbindungen der Formel I von Anspruch 1, dadurch gekennzeichnet, dass der Phenylrest in Formel I in 3,4,5-Stellung trisubstituiert ist.

3. Verbindungen der Formel I, die aus der Gruppe
2-[3,5-Dichlor-4-(4-methylsulfinyl-phenoxy)-phenyl]-hexahydro-1,2,4-triazin-3,5-dion
2-[3,5-Dimethyl-4-(4-methylthio-phenoxy)-phenyl]-hexahydro-1,2,4-triazin-3,5-dion
2-[3,5-Dichlor-4-(3-methyl-4-methylthiophenoxy)-phenyl]-hexahydro-1,2,4-triazin-3,5-dion
2-[3,5-Dichlor-4-(4-chlorphenylthio)-phenyl]-hexahydro-1,2,4-triazin-3,5-dion
2-[3,5-Dichlor-4-(4-methylthio-phenoxy)-phenyl]-hexahydro-1,2,4-triazin-3,5-dion
2-[3,5-Dichlor-4-(4-methylsulfonyl-phenoxy)-phenyl]-hexahydro-1,2,4-triazin-3,5-dion
ausgewählt sind.

4. Verbindungen der Formel I, die aus der Gruppe 2-[3,5-Dichlor-4-(4-methylsulfinyl-phenoxy)-phenyl]-hexahydro-1,2,4-triazin-3,5-dion und
2-[3,5-Dichlor-4-(4-methylsulfonyl-phenoxy)-phenyl]-hexahydro-1,2,4-triazin-3,5-dion ausgewählt sind.

5. Verfahren zur Herstellung von Verbindungen der Formel I gemäss einem oder mehreren der Ansprüche 1 bis 4, dadurch gekennzeichnet, dass man ein substituiertes 1,2,4-Triazin-3,5-(2H,4H)-dion der Formel

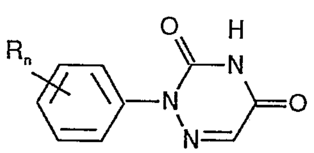

(II),

worin n und R die zu Formel I in den Ansprüchen 1 bis 4 gegebenen Bedeutungen haben, selektiv an der C-N-Doppelbindung hydriert.

6. Verfahren nach Anspruch 5, dadurch gekennzeichnet, dass die Reduktion mit Zink in Eisessig oder Zinn-(II)-chlorid in Salzsäure, gegebenenfalls in einem inerten Lösungs- oder Verdünnungsmittel erfolgt.

7. Verfahren nach Anspruch 5 oder 6, dadurch gekennzeichnet, dass die Reduktion in einem Temperaturbereich von etwa 50 bis 150 °C, vorzugsweise von 80 bis 120 °C durchgeführt wird.

8. Tierarzneimittel, dadurch gekennzeichnet, dass es eine Verbindung der Formel I gemäss einem oder mehreren Ansprüche 1 bis 4 enthält oder aus ihr besteht.

9. Verwendung einer Verbindung der Formel I gemäss Ansprüchen 1 bis 4 zur Herstellung eines Präparates zur Bekämpfung von Protozoenerkrankungen, insbesondere von Coccidiose.

10. Verwendung gemäss Anspruch 9, dadurch gekennzeichnet, dass das Präparat ein Tierfutter ist, das einen Wirkstoff der Formel I in einer Konzentration von 0,1 bis 300 ppm, vorzugsweise 0,5 bis 50 ppm enthält.

**Patentansprüche für den Vertragsstaat AT**

1. Verfahren zur Herstellung von substituierten 2-Phenyl-hexahydro-1,2,4-triazin-3,5-dionen der Formel I

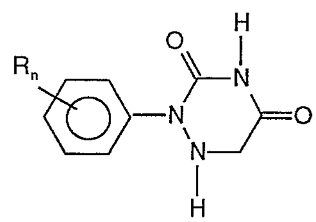

(I),

worin n = zwei oder drei und die einzelnen Substituenten
R = a) Wasserstoff, Chlor, $(C_1-C_6)$-Alkyl, $(C_1-C_6)$-Alkoxy, $(C_1-C_6)$-Alkylthio, $(C_1-C_6)$-Alkylsulfinyl, $(C_1-C_6)$-Alkylsulfonyl oder
b) einen jeweils $R_{a)}$-substituierten Phenoxy-, Phenylthio-, Phenylsulfinyl- oder Phenylsulfonylrest bedeuten und wenigstens ein R ein vorstehend unter b) definierter Substituent ist, sowie deren Alkali-, Erdalkali- oder Ammoniumsalze, dadurch gekennzeichnet, dass man ein substituiertes 1,2,4-Triazin-3,5-(2H,4H)-dion der Formel II

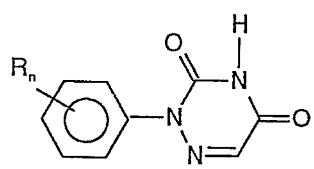

(II),

selektiv an der C-N-Doppelbindung hydriert.

2. Verfahren gemäss Anspruch 1, dadurch gekennzeichnet, dass der Phenylrest in Formel I in 3,4,5-Stellung trisubstituiert ist.

3. Verfahren nach Anspruch 1, dadurch gekennzeichnet, dass die Verbindungen der Formel I aus der Gruppe 2-[3,5-Dichlor-4-(4-methylsulfinyl-phenoxy)-phenyl]-hexahydro-1,2,4-triazin-3,5-dion
2-[3,5-Dimethyl-4-(4-methylthio-phenoxy)-phenyl]-hexahydro-1,2,4-triazin-3,5-dion
2-[3,5-Dichlor-4-(3-methyl-4-methylthiophenoxy)-phenyl]-hexahydro-1,2,4-triazin-3,5-dion
2-[3,5-Dichlor-4-(4-chlorphenylthio)-phenyl]-hexahydro-1,2,4-triazin-3,5-dion
2-[3,5-Dichlor-4-(4-methylthio-phenoxy)-phenyl]-hexahydro-1,2,4-triazin-3,5-dion
2-[3,5-Dichlor-4-(4-methylsulfonyl-phenoxy)-phenyl]-hexahydro-1,2,4-triazin-3,5-dion ausgewählt sind.

4. Verfahren nach Anspruch 1, dadurch gekennzeichnet, dass als Verbindungen der Formel I die Wirkstoffe aus der Gruppe 2-[3,5-Dichlor-4-(4-methylsulfinyl-phenoxy)-phenyl]-hexahydro-1,2,4-triazin-3,5-dion und 2-[3,5-Dichlor-4-(4-methylsulfonyl-phenoxy)-phenyl]-hexahydro-1,2,4-triazin-3,5-dion ausgewählt sind.

5. Verfahren nach einem oder mehreren der Ansprüche 1 bis 4, dadurch gekennzeichnet, dass die Reduktion mit Zink in Eisessig oder Zinn-(II)-chlorid in Salzsäure, gegebenenfalls in einem inerten Lösungs- oder Verdünnungsmittel erfolgt.

6. Verfahren nach einem oder mehreren der Ansprüche 1 bis 5, dadurch gekennzeichnet, dass die Reduktion in einem Temperaturbereich von etwa 50 bis 150 °C, vorzugsweise von 80 bis 120 °C durchgeführt wird.

7. Verwendung einer Verbindung der Formel I gemäss Ansprüchen 1 bis 4 zur Herstellung eines Tierfutters zur Bekämpfung von Protozoenerkrankungen, insbesondere von Coccidiose, wobei das Tierfutter den Wirkstoff der Formel I in einer Konzentration von 0,1 bis 300 ppm, vorzugsweise 0,5 bis 50 ppm enthält.

**Claims for the contracting states: BE, CH, DE, FR, GB, IT, LI, LU, NL, SE**

1. A substituted 2-phenyl-hexahydro-1,2,4-triazine-3,5-dione of the formula

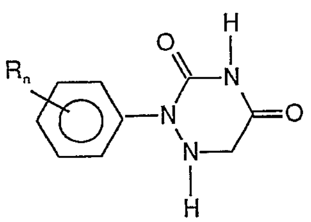

(I),

in which n = two or three and the individual substituents R = a) hydrogen, chlorine, $(C_1-C_6)$-alkyl, $(C_1-C_6)$-alkoxy, $(C_1-C_6)$-alkylthio, $(C_1-C_6)$-alkylsulfinyl or $(C_1-C_6)$-alkylsulfonyl or b) a phenoxy, phenylthio, phenylsulfinyl or phenylsulfonyl radical which is in each case $R_{a)}$-substituted, and at least one R is a substituent defined above under b), and the alkali metal salts, alkaline earth metal salts or ammonium salts thereof.

2. A compound of the formula I of claim 1, wherein the phenyl radical in the formula I is trisubstituted in the 3-, 4- and 5-positions.

3. A compound of the formula I, selected from the group comprising
2-[3,5-dichloro-4-(4-methylsulfinyl-phenoxy)-phenyl]-hexahydro-1,2,4-triazine-3,5-dione,
2-[3,5-dimethyl-4-(4-methylthiophenoxy)-phenyl]-hexahydro-1,2,4-triazine-3,5-dione,
2-[3,5-dichloro-4-(3-methyl-4-methylthiophenoxy)-phenyl]-hexahydro-1,2,4-triazine-3,5-dione,
2-[3,5-dichloro-4-(4-chlorophenylthio)-phenyl]-hexahydro-1,2,4-triazine-3,5-dione,
2-[3,5-dichloro-4-(4-methylthiophenoxy)-phenyl]-hexahydro-1,2,4-triazine-3,5-dione,
2-[3,5-dichloro-4-(4-methylsulfonyl-phenoxy)-phenyl]-hexahydro-1,2,4-triazine-3,5-dione.

4. A compound of the formula I, selected from the group comprising
2-[3,5-dichloro-4-(4-methylsulfinyl-phenoxy)-phenyl]-hexahydro-1,2,4-triazine-3,5-dione and
2-[3,5-dichloro-4-(4-methylsulfonyl-phenoxy)-phenyl]-hexahydro-1,2,4-triazine-3,5-dione.

5. A process for the preparation of a compound of the formula I as claimed in one or more of claims 1 to 4, which comprises selectively hydrogenating a substituted 1,2,4-triazine-3,5-(2H,4H)-dione of the formula

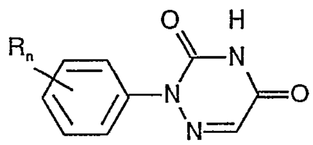

(II),

in which n and R are as defined for formula I in claims 1 to 4, at the C–N double bond.

6. The process as claimed in claim 5, wherein the reduction is carried out with zinc in glacial acetic acid or tin(II) chloride in hydrochloric acid, if appropriate in an inert solvent or diluent.

7. The process as claimed in claim 5 or 6, where in the reduction is carried out within a temperature range from about 50 to 150 °C, preferably from 80 to 120 °C.

8. A veterinary drug, which comprises a compound of the formula I as claimed in one or more of claims 1 to 4, or is composed thereof.

9. The use of a compound of the formula I as claimed in claims 1 to 4 for the preparation of a product for controlling protozoal diseases, in particular coccidiosis.

10. The use as claimed in claim 9, wherein the product is an animal fodder which contains an active compound of the formula I in a concentration of 0.1 to 300 ppm, preferably 0.5 to 50 ppm.

**Claims for the contracting state AT**

1. A process for the preparation of a substituted 2-phenyl-hexahydro-1,2,4-triazine-3,5-dione of the forr la I

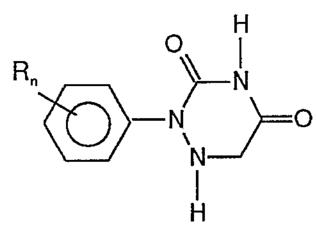

(I),

in which n = two or three and the individual substituents
R = a) hydrogen, chlorine, $(C_1-C_6)$-alkyl, $(C_1-C_6)$-alkoxy, $(C_1-C_6)$-alkylthio, $(C_1-C_6)$-alkylsulfinyl or $(C_1-C_6)$-alkylsulfonyl or b) a phenoxy, phenylthio, phenylsulfinyl or phenylsulfonyl radical which is in each case $R_{a)}$-substituted, and at least one R is a substituent defined above under b), and the alkali metal salts, alkaline earth metal salts or ammonium salts thereof, which comprises selectively hydrogenating a substituted 1,2,4-triazine-3,5-(2H,4H)-dione of the formula II

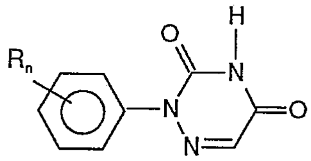

(II),

at the C–N double bond.

2. The process as claimed in claim 1, wherein, in the formula I, the phenyl radical is trisubstituted in the 3-, 4- and 5-positions.

3. The process as claimed in claim 1, wherein the compound of the formula I is selected from the group comprising
2-[3,5-dichloro-4-(4-methylsulfinyl-phenoxy)-phenyl]-hexahydro-1,2,4-triazine-3,5-dione,
2-[3,5-dimethyl-4-(4-methylthiophenoxy)-phenyl]-hexahydro-1,2,4-triazine-3,5-dione,
2-[3,5-dichloro-4-(3-methyl-4-methylthiophenoxy)-phenyl]-hexahydro-1,2,4-triazine-3,5-dione,
2-[3,5-dichloro-4-(4-methylthiophenoxy)-phenyl]-hexahydro-1,2,4-triazine-3,5-dione,
2-[3,5-dichloro-4-(4-chlorophenylthio)-phenyl]-hexahydro-1,2,4-triazine-3,5-dione.

4. The process as claimed in claim 1, wherein an active compound from the group comprising
2-[3,5-dichloro-4-(4-methylsulfinyl-phenoxy)-phenyl]-hexahydro-1,2,4-triazine-3,5-dione
and
2-[3,5-dichloro-4-(4-methylsulfonyl-phenoxy)-phenyl]-hexahydro-1,2,4-triazine-3,5-dione,
is selected as the compound of the formula I.

5. The process as claimed in one or more of clains 1 to 4, wherein the reduction is carried out with zinc in glacial acetic acid or tin(II) chloride in hydrochloric acid, if appropriate in an inert solvent or diluent.

6. The process as claimed in one or more of claims 1 to 5, wherein the reduction is carried out within a temperature range from about 50 to 150 °C, preferably from 80 to 120 °C.

7. The use of a compound of the formula I as claimed in claims 1 to 4 for the preparation of an animal fodder for controlling protozoal diseases, in particular coccidiosis, the animal fodder containing an active compound of the formula I in a concentration of 0.1 to 300 ppm, preferably 0.5 to 50 ppm.

**Revendications pour les Etats Contractants BE, CH, DE, FR, GB, IT, LI, LU, NL, SE**

1. 2-phényl-hexahydro-1,2,4-triazine-3,5-diones substituées, de formule

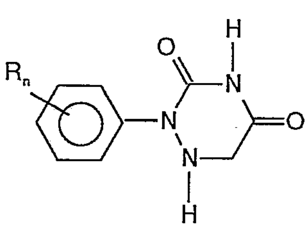

(I),

dans laquelle n = 2 ou 3 et les substituants individuels R représentent a) un atome d'hydrogène ou de chlore ou un groupe alkyle en $C_1$–$C_6$, alcoxy en $C_1$–$C_6$, alkyl($C_1$–$C_6$)-thio, alkyl($C_1$–$C_6$)-sulfinyle, alkyl($C_1$–$C_6$)-sulfonyle, ou b) un radical phénoxy, phénylthio, phénylsulfinyle ou phénylsulfonyle substitué dans chaque cas par $R_{a)}$, et au moins un radical R est un substituant défini ci-dessus en b), et sels alcalins, alcalino-terreux ou d'ammonium de celles-ci.

2. Composés de formule I selon la revendication 1, caractérisé en ce que le radical phényle dans la formule I est trisubstitué en position 3, 4, 5.

3. Composés de formule I, choisis parmi la
2-[3,5-dichloro-4-(4-méthylsulfinyl-phénoxy)-phényl]-hexahydro-1,2,4-triazine-3,5-dione,
la 2-[3,5-diméthyl-4-(4-méthylthio-phénoxy)-phényl]-hexahydro-1,2,4-triazine-3,5-dione,
la 2-[3,5-dichloro-4-(3-méthyl-4-méthylthio-phénoxy)-phényl]-hexahydro-1,2,4-triazine-3,5-dione,
la 2-[3,5-dichloro-4-(4-chlorophénylthio)-phényl]-hexahydro-1,2,4-triazine-3,5-dione,
la 2-[3,5-dichloro-4-(4-méthylthio-phénoxy)-phényl]-hexahydro-1,2,4-triazine-3,5-dione,
la 2-[3,5-dichloro-4-(4-méthylsulfonyl-phénoxy)-phényl]-hexahydro-1,2,4-triazine-3,5-dione.

4. Composés de formule I, choisis parmi la
2-[3,5-dichloro-4-(4-méthylsulfinyl-phénoxy)-phényl]-hexahydro-1,2,4-triazine-3,5-dione et la
2-[3,5-dichloro-4-(4-méthylsulfonyl-phénoxy)-phényl]-hexahydro-1,2,4-triazine-3,5-dione.

5. Procédé pour la préparation de composés de formule I selon une ou plusieurs des revendications 1 à 4, caractérisé en ce que l'on soumet à une hydrogénation sélective au niveau de la double liaison C=N une 1,2,4-triazine-3,5-(2H,4H)-dione substituée de formule

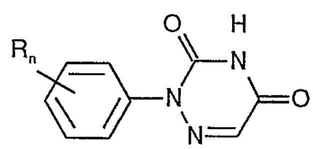

(II),

dans laquelle n et R ont les significations données à propos de la formule I dans les revendications 1 à 4.

6. Procédé selon la revendication 5, caractérisé en ce que l'on effectue la réduction avec du zinc dans de l'acide acétique glacial ou avec du chlorure stanneux dans de l'acide chlorhydrique, éventuellement dans un solvant ou diluant inerte.

7. Procédé selon la revendication 5 ou 6, caractérisé en ce que l'on effectue la réaction dans une gamme de températures d'environ 50 à 150 °C, de préférence de 80 à 120 °C.

8. Médicament à usage vétérinaire, caractérisé en ce qu'il contient un composé de formule I selon une ou plusieurs des revendications 1 à 4, ou consiste en celui-ci.

9. Utilisation d'un composé de formule I selon les revendications 1 à 4, pour la préparation d'une

composition pour la lutte contre des protozooses, en particulier la coccidiose.

10. Utilisation selon la revendication 9, caractérisée en ce que la composition est un aliment pour animaux qui contient une substance active de formule I, à une concentration de 0,1 à 300 ppm, de préférence de 0,5 à 50 ppm.

**Revendications pour l'Etat Contractant AT**

1. Procédé pour la préparation de 2-phényl-hexahydro-1,2,4-triazine-3,5-diones substituées, de formule I

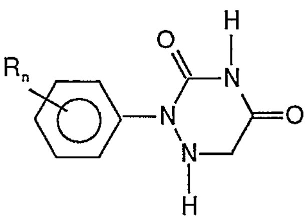

(I),

dans laquelle n = 2 ou 3 et les substituants individuels R représentent a) un atome d'hydrogène ou de chlore ou un groupe alkyle en $C_1$–$C_6$, alcoxy en $C_1$–$C_6$, alkyl($C_1$–$C_6$)-thio, alkyl ($C_1$–$C_6$)-sulfinyle, alkyl($C_1$–$C_6$)-sulfonyle, ou b) un radical phénoxy, phénylthio, phénylsulfinyle ou phénylsulfonyle substitué dans chaque cas par $R_{a)}$, et au moins un radical R est un substituant défini c-dessus en b), ainsi que de leurs sels alcalins, alcalino-terreux ou d'ammonium, caractérisé en ce que l'on soumet à une hydrogénation sélective au niveau de la double liaison C=N une 1,2,4-triazine-3,5-(2H,4H)-dione substituée de formule II

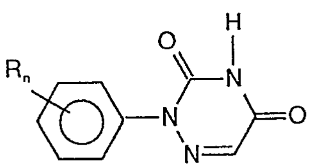

(II),

2. Procédé selon la revendication 1, caractérisé en ce que le radical phényle dans la formule I est trisubstitué en position 3,4,5.

3. Procédé selon la revendication 1, caractérisé en ce que les composés de formule I sont choisis parmi
la 2-[3,5-dichloro-4-(4-méthylsulfinyl-phénoxy)-phényl]-hexahydro-1,2,4-triazine-3,5-dione,
la 2-[3,5-diméthyl-4-(4-méthylthio-phénoxy)-phényl]-hexahydro-1,2,4-triazine-3,5-dione,
la 2-[3,5-dichloro-4-(3-méthyl-4-méthylthio-phénoxy)-phényl]-hexahydro-1,2,4-triazine-3,5-dione,
la 2-[3,5-dichloro-4-(4-chlorophénylthio)-phényl]-hexahydro-1,2,4-triazine-3,5-dione,
la 2-[3,5-dichloro-4-(4-méthylthio-phénoxy)-phényl]-hexahydro-1,2,4-triazine-3,5-dione,
la 2-[3,5-dichloro-4-(4-méthylsulfonyl-phénoxy)-phényl]-hexahydro-1,2,4-triazine-3,5-dione.

4. Procédé selon la revendication 1, caractérisé en ce qu'en tant que composés de formule I, les substances actives sont choisies parmi
la 2-[3,5-dichloro-4-(4-méthylsulfinyl-phénoxy)-phényl]-hexahydro-1,2,4-triazine-3,5-dione et
la 2-[3,5-dichloro-4-(4-méthylsulfonyl-phénoxy)-phényl]-hexahydro-1,2,4-triazine-3,5-dione.

5. Procédé selon une ou plusieurs des revendications 1 à 4, caractérisé en ce que l'on effectue la réduction avec du zinc dans de l'acide acétique glacial ou avec du chlorure stanneux dans de l'acide chlorhydrique, éventuellement dans un solvant ou diluant inerte.

6. Procédé selon une ou plusieurs des revendications 1 à 5, caractérisé en ce que l'on effectue la réaction dans une gamme de températures d'environ 50 à 150°C, de préférence de 80 à 120°C.

7. Utilisation d'un composé de formule I selon les revendications 1 à 4, pour la préparation d'un aliment pour animaux, pour la lutte contre des protozooses, en particulier la coccidiose, dans laquelle l'aliment pour animaux contient la substance active de formule I à une concentration de 0,1 à 300 ppm, de préférence de 0,5 à 50 ppm.